# EUROPEAN PATENT APPLICATION

(11) **EP 3 656 255 A1**
(43) Date of publication of application: **27.05.2020**
(21) Application number: 19209966.1
(22) Date of filing: 19.11.2019
(51) Int. Cl.: A47G 9/10, A61F 5/56

(54) **A PILLOW THAT FACILITATES THE LATERAL SNIFF POSITION, IMPROVED AIRWAY MANAGEMENT AND COMFORT**

(30) Priority: 20.11.2018 US 201862769869 P
(71) Applicant: Popitz, LLC, Weston, MA 02493 (US)
(72) Inventor: POPITZ, Michael D., Marion, MA 2738 (US); FLIGOR, Andrew T., Weston, MA 2493 (US); DRAKE, Jesse S., Westborough, MA 1581 (US); MILLER, Howard, Concord, MA 1742 (US); MCCARTHY, Justin, Boxborough, MA 1719 (US)
(74) Representative: Grünecker Patent- und Rechtsanwälte PartG mbB

(57) **Abstract**

In one aspect, an apparatus for supporting the head and neck of a user for airway management is disclosed, which comprises a top surface including at least one head-receiving portion (112b) configured and dimensioned for receiving and supporting a user's head, and at least one recess neck opening (114a) for supporting a user's neck when the user's head is received in the head-receiving portion (112b). The apparatus further includes at least one chin support (116a) protruding above the top surface and configured for facilitating placing the user in a sniff position when the user's head is received in the head-receiving portion (112b). The chin support (116a) can include a top surface segment and a lateral surface segment, where at least a portion of the lateral surface segment of the chin support (116a) extends from said top surface segment thereof to said at least one recess neck opening.

## Description

### TECHNICAL FIELD

The present disclosure relates to an apparatus (herein also referred to as a pillow or a head-positioning apparatus) for airway management that can provide support for the head and neck of a user while aligning the oropharyngeal, laryngeal, and tracheal axes of the human head and neck for airway management in the lateral decubitus position.

### BACKGROUND

Many attempts have been made to design and improve pillows in order to reduce snoring or other obstructive breathing, and/or to facilitate intubation. Obstructive breathing may occur during sleep, or sedation, most commonly in the supine position due to the effects of gravity on the tongue. Pillows have been developed to reduce airway obstruction in the supine position. For example, U.S. Pat. No. 5,048,136 discloses such a pillow. Sleeping in the lateral decubitus position (wherein person sleeps on their side) has been also shown to help reduce, but not eliminate, obstructive breathing during sleep. Because of this known fact, pillows that facilitate sleeping in the lateral position have been developed to keep the airways open including those disclosed in US. Pat. Nos, 7,908,691 B2 and 8,677,531B2.

As described in U.S. Patent No. 8,677,S31B2, one method of opening the airways includes aligning the oropharyngeal, laryngeal and tracheal axes by placing the patient into the "sniff" position, which has been determined to be the most effective positional method for improving the patency of the airway and therefore enhancing the volume and smoothness of the flow of air or oxygen into the patient and the flow of carbon dioxide out.

However, there are shortcomings with conventional methods of managing air and oxygen flow through an individual's airway. For example, the conventional methods fail to accommodate the following: varying patient morphologies (for example variances in shoulder to neck distance or neck and jaw length), differing mattress compression caused by variations in user weight, and differences in mattress indentation force deflection (a measure of softness or firmness of a pillow or sleep mattress), all of which can prevent a patient from sleeping comfortably. The method described in the referenced patent also allows a sleeping patient to easily move from the lateral decubitus position into the supine position wherein the alignment of the axes of airways is lost and the luminal diameter of the airways is diminished.

Thus a need exists for a pillow that is more effective in promoting restful sleep while aligning and/or increasing the diameter of the upper airways (those proximal to the cartilaginous trachea). This improvement should include features that allow adjustments for differing patient morphologies such as varying neck to shoulder measurements for shoulder depth, for differences in patient weight and mattress deformation (affecting required pillow height), for the use of ventilation devices (such as CPAP or BiPAP),for comfortable disposition of the arm during sleep and for varying the chin angle (head size, patient height, shoulder width, patient weight, chin length, range of motion (or lack thereof), of the patients' neck), thus allowing the patient to sleep in the most comfortable position with optimal alignment of the oropharyngeal, laryngeal, and tracheal axes.

### SUMMARY

Embodiments of the present invention address and overcome one or more of the above shortcomings and drawbacks, by providing methods, systems, and apparatuses for positioning and maintaining the user comfortably into the lateral sniff position.

In one embodiment, an apparatus for supporting the neck and the head of a user for airway management includes a top section with a top surface and a bottom mating surface, where the top surface is located on a side opposite the bottom mating surface; a bottom section with a bottom surface and a top mating surface, the top section mating surface fitting snuggly onto the bottom section mating surface where the indentation deflection load (IDL) of one section can be greater than the IDL of the second section; where the bottom mating surface of the top section and top mating surface of the bottom section fit snugly together through opposing interfaces and where the opposing interfaces are of sufficient height and depth to prevent the sections from sliding or moving relative to one another; where the height of either the top or bottom sections can vary such that differing user neck and shoulder lengths can be accommodated; where a cut-out on a portion of the pillow, parallel to the user's body accommodates the lateral extension and comfortable anatomic positioning of both arms, and especially the dependent arm, of the user.

In some embodiments, the top section of the pillow can include a removable and replaceable segment that can be removed to provide access to the user's mouth and nose, e.g., to accommodate a ventilation device. Further, in some embodiments, the pillow can include a left head-receiving portion and a right head-receiving portion for receiving and supporting a user's head in the left and the right lateral decubitus position. A ridge or raised surface can separate the left head-receiving portion from the right head-receiving portion. The ridge or raised surface between the first and second head-receiving portions assists in preventing the user from inadvertently assuming (or consciously attempting) the supine position while also positioning the user's head into the proper lateral and sniff position or lateral sniff ramp position. In some embodiments, the ridge can have a non-uniform height with a maximum height in a range of about 1 to about 4 inches, e.g., 2.5 inches to about 3.5 inches, relative to the bottom of the head-receiving portions. A left neck supporting surface is connected to the left head-receiving surface and a right neck supporting surface is connected to the right head-receiving surface, where the neck supporting surfaces are dimensioned to support the user's neck.

The top section of the pillow can further include at least one chin support for facilitating the placement of a user in a lateral decubitus position. In some such embodiments, the height of the chin support can vary between 1 inch and 4.5 inches so that the topmost surface of the chin support is not higher than user's ear aperture level to the top of the epicanthus or outer corner of the down side eye, thus preventing claustrophobia tendencies on user's part and/or obstruction of the user's visual axis. The surfaces of both head-receiving portions, the ridge or raised surface between the head-receiving portions, and the chin support can collectively align the user's oropharyngeal, laryngeal and tracheal airways in the sniff lateral position with the user's head received in one of the head-receiving portions. In many embodiments, the chin support has a compound slope on its inner surface which can be the mirror image of the compound slope of the neck and platysmal surface of the side of the user's neck allowing the inner surface to comfortably receive said lateral platysmal surface.

In some embodiments, the recess neck openings (herein also referred to as neck channels) can be angled relative to the shoulder-receiving portions to position the user's head and neck in an anterior direction, thus allowing for lower neck flexion. The chin support and ear aperture triangulate the user to permit the head and upper neck to be in an extension position,

In one embodiment, an apparatus for supporting the neck and head of a user for airway management includes one or more head-receiving portions shaped and dimensioned to support the side of a user's head, a chin support that includes an adjustable chin positioner that can be adjusted to support the chin of a user, and a neck supporting surface shaped and dimensioned to support a side of the user's neck; wherein the depth of the recessed surface corresponding to the user's chin can be approximately 2 inches, the depth of the recessed portion of the head-supporting surface can be approximately 3 inches and the depth of a recessed portion of the head supporting surface corresponding to the back of the user's head can be approximately 3 inches, and where the adjustable chin positioner can allow the user's Occipito-Atlanto-Axial joint to be adjusted between 5° and 30°, e.g., in a range of between 18-24°, which can be clinically significant. This can allow placing the user in the anatomic sniffing position providing greater occipito-atlanto-axial extension compared to simple head extension.

In one aspect, an apparatus for supporting the head and neck of a user for airway management is disclosed, which comprises a top surface including at least one head-receiving portion configured and dimensioned for receiving and supporting a user's head, and at least one recess neck opening for supporting a user's neck when the user's head is received in the head-receiving portion. The apparatus further includes at least one chin support protruding above the top surface and configured for facilitating the placement of the user in a sniff position when the user's head is received in the head-receiving portion. The chin support can include a top surface segment and a lateral surface segment, where at least a portion of the lateral surface segment of the chin support extends from said top surface segment thereof to said at least one recess neck opening,

The head-receiving portion, the recess neck opening and the chin support can be positioned relative to one another and dimensioned such that when the user is in a lateral decubitus position with the head received by the head-receiving portion, oropharyngeal, laryngeal and tracheal axes are substantially aligned. Further, the head-receiving portion, the recess neck opening and the chin support can be positioned relative to one another and dimensioned such that when the user is in a lateral decubitus position with the user's head received by the head-receiving portion, the user's upper cervical spine experiences an extension in a range of about 5 to about 20 degrees and the user's lower cervical spine experiences a flexion in a range of about 5 to about 15 degrees.

In some embodiments, a maximum height difference between the top surface segment of the chin support and the bottom of a respective head-receiving portion is in a range of about 1 inch to about 5 inches, e.g., in a range of about 2 inches to about 4 inches. In many embodiments, the maximum height of the chin support is selected so as not to be higher than the zygomatic arch of a user's facial bones.

In some embodiments, at least a portion of an inner lateral surface of the chin support (e.g., a portion of the lateral surface of the chin support facing a head-receiving portion) exhibits a compound slope that varies along two orthogonal directions. For example, the slope of such a lateral portion of the chin support can show variation in a downward direction toward the head-receiving portion as well as along a direction substantially orthogonal to such a downward direction. Such variations of the slope along any of those directions can be, for example, in a range of about 20 degrees to about 90 degrees.

In some embodiments, the top surface segment of the chin support can be downwardly slanted toward a lateral side of the top surface of the top section of the pillow. In other embodiments, the top surface segment of the chin support can be flat.

In some embodiments, the neck recess opening can be in the form of a curved ridge. In some embodiments, the recess neck opening can be curved and characterized by a varying radius of curvature from one end thereof to the other. By way of example, the radius of curvature of the recess neck opening can vary between about 1 inch to about 4 inches, e.g., in a range of about 2 and 3 inches.

In some embodiments, the apparatus for supporting the head and neck of a user can include a left and a right head-receiving portion, which are separated from one another by a ridge. In some such embodiments, the apparatus can further include a left and a right recess neck opening, which are also separated by the ridge, Further, in some such embodiments, the apparatus includes a left chin support and a right chin support for facilitating the placement of the user in a sniff position when the user's head is received in the left and right head-receiving portions.

In some embodiments, the chin support can further include an adjustable chin positioner that can be moved to adjust the configuration of the chin support for accommodating different users. In other words, the adjustable chin positioner can allow configuring the chin support so that it can accommodate differing user morphologies and place such users in a sniff position.

The adjustable chin positioner can be implemented in a variety of different ways. For example, in some embodiments, the adjustable chin positioner can be implemented by providing an opening in the inner lateral surface of the chin support, which can at least partially extend from the inner lateral surface toward an outer lateral surface of the chin support. The adjustable chin positioner can include a post that is configured to be movably positioned within the opening formed in inner lateral surface of the chin support. The post can extend between a proximal surface and a distal surface. A portion of the lateral surface of the chin support surrounding the opening can be recessed relative to the opening to allow the post to swivel about the opening. When the post is fully engaged within the opening, the proximal surface of the post is substantially flush with the inner lateral surface of the chin support. In some embodiments, the proximal surface of the post has a compound curvature that in combination with the rest of the lateral surface of the chin support provides a suitable compound curvature for comfortably positioning a user in a sniff position. In some such embodiments, the compound curvature of the proximal surface of the post can complement the compound curvature of the lateral surface of the chin support such that when the post is fully engaged within the opening with the proximal surface thereof substantially flush with the inner lateral surface of the chin support, the combination of the proximal surface of the post and the remainder of the inner lateral surface of the chin support forms a substantially contiguous surface.

As noted above, the post can be moved in and out of, and/or swivel about, the opening provided in the chin support to provide adjustments for accommodating different users. In some embodiments, a plurality of ridges can be provided on the inner surface of the opening to provide multiple settings for the extension of the post outside of the opening.

In some embodiments, the adjustable chin positioner can be implemented by providing a groove on a surface of the chin support, e.g., on or in proximity of the top surface segment of the chin support, where the adjustable chin positioner includes a chin support block that can be movably engaged with the groove to move back-and-forth along the groove. In some such embodiments, the bottom surface of the block comprises a sawtooth surface and the groove includes a mating sawtooth surface for engaging with the sawtooth surface of the block. In some embodiments, the groove can have a curved profile, e.g., it can extend from a front end of the top surface to a lateral side thereof.

In some embodiments, the ridge separating the right and the left head-receiving portions can be configured to inhibit inadvertent transitioning of a user from a lateral decubitus position to a supine position. For example, in some such embodiments, the ridge can have a maximum height in a range of about 1 inch to about 5 inches. In some embodiments, the ridge can have a non-uniform height. Such non-uniformity of the ridge's height can be selected so as to inhibit a user from moving from a lateral decubitus position to a supine position while ensuring that the ridge would not make the user uncomfortable. In some such embodiments, the ridge exhibits a greater height proximate a front side of the top surface relative to a backside thereof. For example the ridge can exhibit a height non-uniformity in a range of about 10% to about 300%.

In some embodiments, the maximum width of the ridge, which can be defined as the width of the portion of the ridge extending between the left and the right recess neck openings, can be, for example, in a range of about 3 inches to about 6 inches, e.g., in a range of about 4 to about 5 inches.

In some embodiments, each of the head-receiving portions has a downward-sloping surface, which extends from a top edge of the head-receiving portion to a bottom end thereof. In some embodiments, the surface of the head-receiving portion exhibits a varying slope across different segments thereof In some such embodiments, the surface of the head-receiving portion can exhibit a compound slope characterized by variations of the slope along two orthogonal directions. For example, the slope of such a head-receiving surface can vary along a downward direction and also along a direction that is substantially orthogonal to the downward direction. In some other embodiments, the slope variation of the surface of a head-receiving portion can be only along one direction. Further, in other embodiments, a head-receiving portion may exhibit a single slope across the entire surface thereof.

By way of example, in some embodiments in which the apparatus includes a left head-receiving portion and a right head-receiving portion, the left head-receiving portion comprises a first segment positioned proximate a left side of said top surface, a second segment positioned proximate a back side of said top surface, a third segment positioned proximate said ridge, and a fourth segment positioned proximate a front side of said top surface, where the third segment exhibits a steeper slope relative to the second segment, and the second segment exhibits a steeper slope relative to the first segment. Further, in some such embodiments, the right head-receiving portion comprises a first surface segment positioned proximate a left side of the top surface, a second surface segment positioned proximate a back side of the top surface, a third surface segment positioned proximate said ridge, and a fourth surface segment positioned proximate a front end of said surface, where the third surface segment exhibits a slope steeper than that of the second surface segment and said second surface segment exhibits a slope steeper than that of the first segment. In some embodiments, the variation of the slope across the surface of a head-receiving portion can be, for example, in a range of about 20 degrees to about 90 degrees,

In some embodiments, the apparatus for supporting the head and neck of a user for airway management can include at least one ear opening (also herein referred to as ear hole) that is disposed in at least one of the head-receiving portions, e.g., at the bottom of the head-receiving portion. The ear hole can be configured and dimensioned to at least partially receive a user's ear while the user is in a lateral decubitus position with the user's head received and supported in the head-receiving portion. By way of example, the ear hole can have a maximum cross-sectional dimension, e.g., a diameter when the cross-sectional profile is circular, in a range of about 1 to about 5 inches, e.g., in a range of about 2 inches to about 4 inches. The ear hole can have a variety of different cross-sectional profiles, such as, circular, elliptical, polygonal, etc. By way of example, the ear hole can have a substantially cylindrical profile. In some such embodiments, the lateral surface of each ear hole can exhibit a convex profile. The ear hole can extend from the top surface to an opposed bottom surface of the apparatus.

In some embodiments, the ear hole is positioned and dimensioned so as to muffle noise generated by the apparatus, e.g., due to compression of the apparatus by a user's head, which is experienced by the user while the user's head is maintained in said at least one head-receiving portion.

In some embodiments, rather than an ear hole, the apparatus can include an indentation at the bottom of a head-receiving portion for accommodating at least a portion of a user's ear.

In some embodiments, at least a portion of a lateral surface of the ear hole can be covered with a ventilation material. By way of example, the ventilation material can be in the form of a mesh. Some examples of suitable ventilation materials include, without limitation, silk, cotton, wool, polyester or combinations thereof.

In some embodiments, the top surface comprises at least one removable and replaceable portion such that removal of the removable portion allows access to the user's nose and mouth when the user's head is received in said at least one head-receiving portion in a lateral decubitus position. By way of example, the removable portion can be positioned between the head-receiving portion and a lateral side of the top surface. By way of example, the removal of the removable portion can allow the user to use a monitoring and/or diagnostic device, such as a CPAP device, while using the apparatus in a lateral decubitus position.

In some embodiments, the apparatus can further include a cover for at least partially enclosing the apparatus. In some such embodiments, a portion of the cover can extend through the ear hole to be fastened to another portion of the cover for securing the cover to the apparatus.

An apparatus according to the present teachings can be fabricated using any suitable material, including a variety of different polymeric materials. Some examples of such materials include, without limitation, polyurethane, latex polyurethane, viscoelastic polyurethane, memory foam, polyethylene, and EVA (ethylene-vinyl acetate), among others. In some embodiments, the apparatus is formed of a foamed material. In some embodiments, the density of the foamed material from which the apparatus is formed can be, for example, in a range of about 1.5 to about 5 pound/ft³.

In a related aspect, an apparatus for supporting the head and neck of a user for airway management is disclosed, which comprises a top section, and a bottom section configured for removably and replaceably engaging with said top section. The top section includes a left portion separated from a right portion by a ridge. Each of the left and right portions includes a head-receiving cavity for receiving and supporting a user's head, a recess neck opening for supporting a user's neck when the user's head is received in said head-receiving cavity, and a chin support for facilitating the placement of the user in a sniff position when the user's head is received in said head-receiving cavity in a lateral decubitus position.

In some embodiments, the head-receiving cavity, the recess neck opening and the chin support of each of the right and left portions are positioned relative to one another and dimensioned such that when the user is in a lateral decubitus position with the user's head received by the head-receiving portion, the user's oropharyngeal, laryngeal and tracheal axes are substantially aligned. Further, in some embodiments, the head-receiving cavity, the recess neck opening and the chin support of each of the left and right portions are positioned relative to one another and dimensioned such that when the user is in a lateral decubitus position with the user's head received by the head-receiving cavity, the user's upper cervical spine experiences an extension in a range of about 5 to about 20 degrees and the user's lower cervical spine experiences a flexion in a range of about 5 to about 15 degrees,

The top section can include a top surface, a bottom surface opposed to said top surface, a front surface, a back surface opposed to said front surface, a left side surface, and a right side surface. Further, the bottom section can include a top surface shaped to matingly engage with the bottom surface of the top section, and a bottom surface opposed to said top surface,

The top surface of the top section includes the head-receiving cavities, the recess neck openings and the chin supports. In some embodiments, each recess neck opening can be in the form of a ridge that extends from a respective chin support to the ridge separating the left and right portions of the top section.

In some embodiments, at least one of said head-receiving cavities includes an indentation at a bottom end thereof for receiving at least a portion of a user's ear when the user's head is received in said head-receiving cavity in a lateral decubitus position. In some other embodiments, at least one of the head-receiving cavities includes an ear hole for receiving at least a portion of a user's ear. In some embodiments, such an ear hole extends from the top surface of the top section to the bottom surface thereof.

In some embodiments, the bottom section includes two ear holes that extend from a top surface to a bottom surface thereof, where the ear holes in the bottom section are positioned so as to be substantially aligned with the ear holes formed in the top section upon engagement of the top section with the bottom section, where "substantially aligned" as used herein with reference to the ear holes in the top and the bottom sections means that the planes of the inner surfaces of the ear holes in the top and the bottom sections are aligned to within at least 0.125". In other words, a misalignment of such surfaces, if any, is at most 0.125".

Each of the chin supports protrudes above the top surface of the top section. Further each of the chin supports provides a cavity for receiving a respective protruding element of the bottom section. In some embodiments, each chin support can include a lateral surface segment and a top surface segment, where at least a portion of an inner portion of the lateral surface segment (i.e., at least a portion of the lateral surface segment facing a head-receiving portion) exhibits a compound slope that varies along two orthogonal directions.

In some embodiments, at least one of the left or the right chin support can include an adjustable chin positioner. The adjustable chin positioner can be implemented in a variety of different ways, such as those discussed above.

In some embodiments, the front surface of the top section includes a recess for receiving and supporting a user's shoulder. Each shoulder-receiving recess can extend between a respective chin support and the ridge separating the left and the right portions of the top section. In some embodiments, the ratio of the width to the depth of each shoulder-receiving portion, as defined further below, can be in a range of about 1.5:1 to about 6:1. In some embodiments, the width of each shoulder-receiving section can be, for example, 6 inches to about 18 inches, and the depth of each shoulder-receiving portion can be, for example, in a range of about 1 inch to about 12 inches.

Further, the bottom section can include a respective shoulder-receiving recess such that when the top section is engaged with the bottom section, the top and bottom recesses cooperatively provide shoulder supporting surfaces for left and right lateral decubitus positions.

In some embodiments, the top section exhibits a hardness characterized by an IDL (indentation-deflection-load) value in a range of about 12 to 50, e.g., in a range of about 20 to about 40. Further, in some embodiments, the hardness of the top section is different from the hardness of the bottom section. By way of example, the difference between the hardness of the top and the bottom sections can be at least about 0.5 IDL, e.g., in a range of about 0.5 IDL to about 2 IDL.

In some embodiments, the ridge separating the left portion from the right portion is configured to inhibit involuntary transitioning of the user from a lateral decubitus position with the user's head received in one of said head-receiving cavities into a supine position. In some embodiments, the ridge can include a cavity into which a mating protrusion provided on the top surface of the bottom section can be inserted upon engagement of the bottom section of the apparatus with the top section.

In some embodiments, the recess neck opening and the chin support of at least one of the left and right portions are positioned relative to one another and dimensioned such that if the user moves from a lateral decubitus position to a supine position with the user's occipital lobe within the head-receiving cavity, the user remains in a sniff position.

In some embodiments, at least one of the right and left portions of the top section (and optionally the bottom section of the apparatus) comprises a removable and replaceable segment such that removal of said segment allows access to the user's nose and mouth when the user's head is received in a respective head-receiving cavity in a lateral decubitus position.

In another aspect, an apparatus for supporting the head and neck of a user for airway management is disclosed, which includes a polymeric block having a top surface, a bottom surface, a right surface, a left surface, a front surface and a back surface. At least one head-receiving cavity is formed in said top surface for receiving and supporting a user's head. Further, at least one recess neck opening is provided on said top surface for supporting a user's neck when the user's head is received in said head-receiving cavity. The apparatus further includes a chin support for facilitating the placement of the user in a sniff position when the user's head is received in said head-receiving cavity, where the chin support protrudes above the top surface at a maximum height relative to the bottom of the top surface in a range of about 2 to about 4 inches.

In some embodiments, the head-receiving cavity, the recess neck opening and the chin support are positioned relative to one another and dimensioned such that when the user is in a lateral decubitus position with the head received by the head-receiving cavity, oropharyngeal, laryngeal and tracheal axes are substantially aligned.

In some embodiments, the head-receiving cavity can exhibit a compound slope.

In some embodiments, the above apparatus can include a left head-receiving cavity and a right head-receiving cavity that are separated from one another by a ridge. In some such embodiments, the ridge can have a maximum height in a range of about 1 to about 4 inches. Further, in some such embodiments, the ridge can have a non-uniform height with the height decreasing from a portion proximate the front surface of the apparatus to a portion proximate the back surface of the apparatus.

In some embodiments, the polymeric block can include a portion that is removable and replaceable such that its removal can provide access to a user's mouth and nose while the user is in the lateral decubitus position with the user's head received in the head-receiving cavity.

In some embodiments, the recess neck opening can have a radius of curvature in a range of about 1 inch to about 4 inches.

In some embodiments, the apparatus can include at least one shoulder-receiving recess for receiving and supporting a user's shoulder. In some such embodiments, the shoulder-receiving recess can have a width in a range of about 6 inches to about 18 inches.

Additional features and advantages of the invention will be made apparent from the following detailed description of illustrative embodiments that proceeds with reference to the accompanying drawings,

The accompanying drawings, which are incorporated herein and form part of the specifications, illustrate various embodiments of a pillow for facilitating the lateral sniff position and for facilitating airway management. Together with the descriptions the figures further serve to explain the principles of the pillow described herein and thereby enable a person skilled in the applicable arts to make the apparatus.

### Brief Description of the Drawings

FIGs. **1A** and **1B** schematically depict an individual's airway passages in a supine position as well as in a sniff position, indicating better alignment of the airway passages in the sniff position;
FIG. **2A** is a schematic side view of a pillow according to the present teachings having a top section that is removably engaged with a bottom section;
FIG. **2B** shows schematic top and side views of the top section of the pillow depicted in FIG. **2A**;
FIGs. **2C** and **2D** depict schematic views of the top surface of the top section of the pillow depicted in FIGs. **2A** and **2B**;
FIG. **2E** schematically depicts the bottom surface of the top section of the pillow depicted in FIGs. **2A** and **2B**;
FIG. **3A** depicts a partial view of the top section of the pillow;
FIGs. **3B-3C** schematically depict that the lateral surface of the chin support according to an embodiment has a compound slope;
FIG. **4** schematically depicts a ventilation material coupled to a recessed ear hole formed in a head-receiving portion of a pillow according to the present teachings;
FIGs. **5A**, **5B**, **5C**, and **5D** depict various schematic views of a pillow according to an embodiment depicting relationship of various elements of the pillow by utilizing a plurality of putative planes;
FIG. **6A** schematically depicts the bottom surface of the bottom section of the pillow depicted in FIG. **2A**;
FIG. **6B** schematically depicts the top surface of the bottom section of the pillow depicted in FIG. **2A**;
FIG. 7 schematically depicts an individual using a pillow according to an embodiment in the left lateral decubitus position;
FIG. **8** schematically depicts an individual using a pillow according to an embodiment in the right lateral decubitus position in a sniff position, indicating better alignment of the airway passages relative to a neutral position;
FIG. **9A** schematically depicts the top section of a pillow according to an embodiment in which a left and a right adjustable chin positioner are coupled to the left and right chin supports of the pillow;
FIGs. **9B** and **9C** schematically depict an example of an implementation of an adjustable chin positioner in which a chin support block is movably engaged: within a groove provided on a surface of the chin support;
FIGs. **10A**, **10B**, and **10C** schematically depict another example of an adjustable chin positioner according to another embodiment;
FIG. **11A** is a partial schematic view of a pillow according to an embodiment, which includes a removable and replaceable block;
FIGs. **11B** and **11C** schematically depict the removable block shown in FIG. **11A** includes two grooves for movably coupling to two rails provided on the top section of the pillow;
FIG. **12** schematically depicts a pillow according to an embodiment of the present teachings that includes a left and a right arm cut-out;
FIGs. **13A**, **13B**, and **13C** schematically depict a pillow according to an embodiment enclosed within a pillow cover;
FIGs. **14A**, **14B**, and **14C** schematically depict a pillow according to an embodiment enclosed within another pillow cover, and
FIG. **15** schematically depicts a pillow according to an embodiment, which is formed of a single polymeric piece.

### Detailed Description

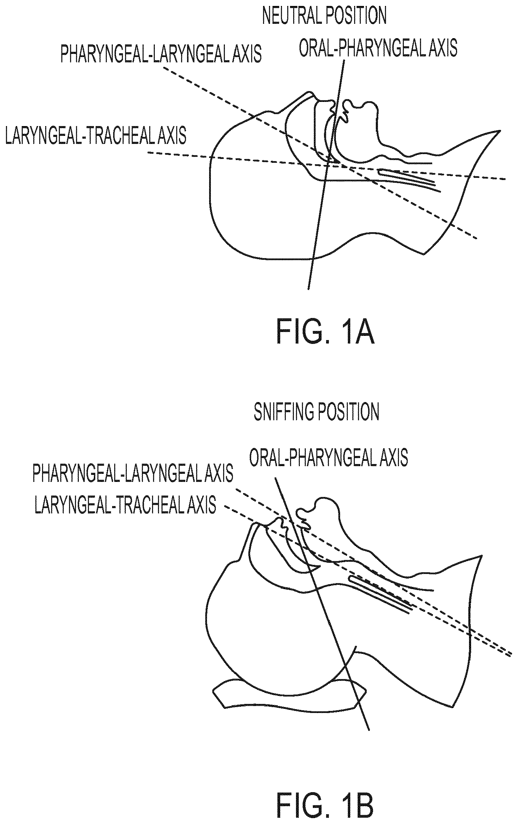

The present invention is generally directed to an apparatus for supporting the head and neck of a user for airway management. In many embodiments, an apparatus according to the present teachings can be used to place a user in a position in which the user's oropharyngeal, laryngeal and tracheal axes are substantially aligned, as discussed in more detail below. With reference to FIGs. **1A** and **1B**, in a neutral position, e.g., when a person is lying on his/her back on a flat surface with the occipital portion of the skull supported by that surface, the pharyngeal and laryngeal axes are not aligned and the oral axis is substantially normal to the supporting surface and can typically form an angle greater than about 80 degrees with the pharyngeal and laryngeal axes. In contrast, as shown schematically in FIG. **1B**, in a so-called "sniffing" or "sniff" position, the pharyngeal and the laryngeal axes can be substantially aligned. The term "substantially aligned" as used herein in reference to airway axes, means a variation from perfect parallelism by at most 10 degrees. Further, the oral axis can be better aligned with the pharyngeal and laryngeal axes compared to the neutral position. For example, in a sniff position, the angle between the oral axis and any of the pharyngeal and/or laryngeal axes can be in a range of about 5 degrees to about 30 degrees.

Various terms as used herein have their ordinary meanings. As noted above, the term "substantially aligned," as used herein with reference to the airway axes, refers to an alignment that may deviate from a state of perfect alignment by at most 10 degrees. The term "about," as used herein, indicates a deviation of a numerical value by at most +/- 10 percent.

Reference will be made in detail to embodiments of the present invention with reference to the accompanying figures, in which like reference numerals will indicate like elements. While specific configurations are discussed it should be noted that this is for illustrative purposes. The present invention relates to an apparatus (herein also referred to as a pillow, a head-positioning device or a head-positioning apparatus) for aligning the oropharyngeal, laryngeal, and tracheal axes and the extension of the Occipito-Atlanto-Axial joint, together with flexion of the lower cervical spine for airway management while the user is in the lateral decubitus position. Airway management involves adjusting the patient head and neck for improved ventilation and respiration. By improving the position of a user's head and neck, the user can experience improved sleep, rest, oxygenation and ventilation and avoid airway obstruction and airflow turbulence that may result, for example, in snoring, Although a pillow has been introduced to align the upper airways of the human head and neck while in the lateral decubitus position, it has certain shortcomings, which the current invention addresses. For example, a pillow according to the present teachings can accommodate differing user morphologies (for example neck to shoulder distance, which can vary widely even for individuals of the same height and weight), mattress compression (which varies with indentation force load and user's weight). Further, in some embodiments, a pillow according to the present teachings provides at least one arm cutout to allow disposition of the user's arm. Some embodiments are directed to pillows that can be used during sleep, while others are directed to pillows that can support the individual's neck during other activities.

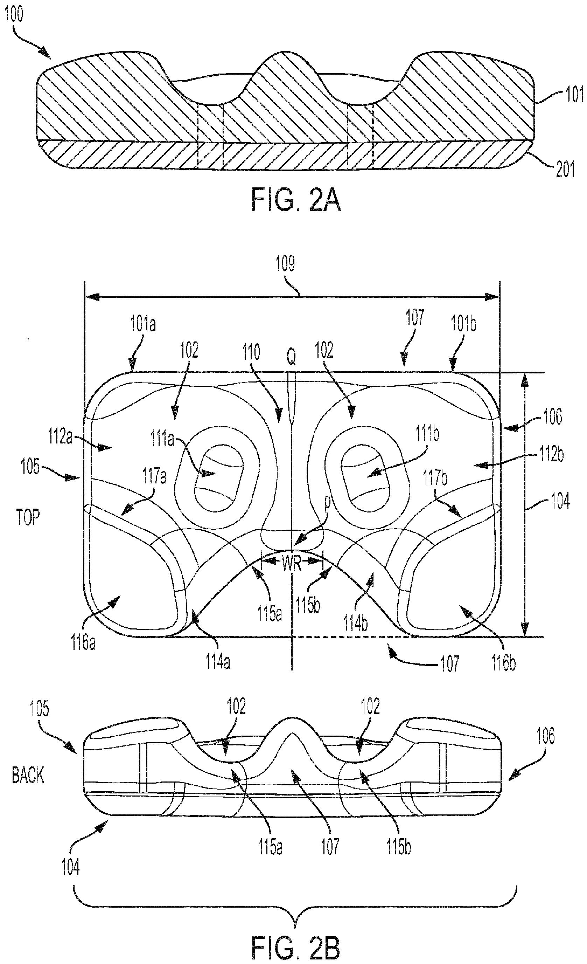

With reference to FIGs **2A**, **2B**, a pillow **100** according to one embodiment of the present invention is disclosed, which allows left and right side sleeping for a user. The sizes of various features of the pillow **100** can be adjusted to accommodate many users' heights and weights. By way of example, the pillow can be sized for use by a 5'8", 180 lb. male or an average user. One of ordinary skill will appreciate that the dimensions of various features of the pillow **100** can be adjusted based on the present teachings to optimize the pillow for larger and smaller users.

In this embodiment, the pillow **100** includes a top section **101** and a bottom section **201**, which can matingly engage with the top section **101** in a manner discussed in more detail below. The top section **101** includes a left portion (herein also referred to as left segment) **101a** and a right portion (herein also referred to as right segment) **101b**, which are separated from one another by a ridge or raised surface **110.** As discussed in more detail below, the left portion **101a** and the right portion **101b** accommodate left and right lateral decubitus positions of the user. Further, as discussed in more detail below, the ridge or raised surface **110** can assist in aligning a user's head and prevent the user from inadvertently assuming the supine position.

In this embodiment, the top section **101**, which has a generally rectangular cross-sectional profile, extends axially from a top surface **102** to a bottom surface **104.** Further, the top section **101** extends laterally along a width dimension **109** from a left side surface **105** to a right side surface **106** and along a length dimension **104** from a front surface **107** to a back surface **108.**

In some embodiments, the height of the top section **101** of the pillow, defined as the maximum distance between its top surface **102** and its bottom surface **104** in uncompressed condition of the pillow, can be, for example, in a range of about 2 inches to about 6 inches, e.g., less than about 5.5 inches. Further, in some embodiments, the width dimension **109** and the length dimension **104** of the top section **101** can vary based on a population of users for whom the pillow is designed. By way of example, for the average user the length dimension **104** is about 16 inches and the width dimension **109** is about 27 inches.

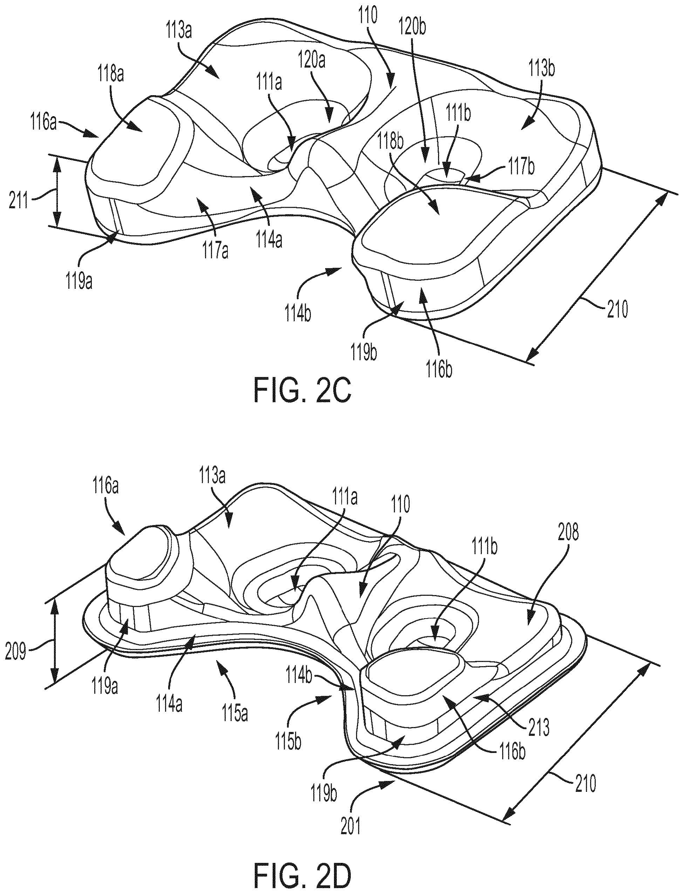

With reference to FIGs. **2A**, **2B**, **2C** and **2D**, the top surface **102** is configured and dimensioned to accept the head and neck of a user in the right and left decubitus lateral positions. In particular, the top surface **102** includes a right head-receiving portion **112a** and a left head-receiving portion **112b** for receiving and supporting a user's head in the right and left lateral decubitus positions.

The top section **101** further includes left and right recess neck openings **114a/114b** and left and right shoulder cut-outs **115a/115b** for receiving and supporting a user's shoulder in left and right decubitus positions, respectively. In this embodiment, the shoulder cut-outs **115a/115b** (herein also referred to as shoulder-receiving portions) are formed as curved portions of the front surface **107** of the top section. Further, the left and the right recess neck openings **114a/114b** are in the form of curved ridges disposed, respectively, over the shoulder receiving portions (herein also referred to as shoulder cut-outs) **115a/115b**.

In this embodiment, the head-receiving portions **112a/112b** are formed by surface portions **113a** and **113b** of the top surface **102**, respectively. The left head-receiving surface portion **113a** is in the form of a downward-sloping surface that is circumscribed by a portion of the left side surface **105**, a portion of the back surface **108**, the inner lateral surface of a right chin support **116a** (which is discussed in more detail below), the left recess neck opening **114a**, and the ridge **110.** Further, the surface **113b** of the right head-receiving portion **112b** is also in the form of a downward-sloping surface that is circumscribed by a portion of the right side surface **106**, a portion of the back surface **108,** the inner lateral surface of a left chin support **116b** (which is also discussed in more detail below), the right recess neck opening **114b** and the ridge **110.** In this embodiment, the head-receiving surface portions **113a** and **113b** terminate at bottom at recessed ear openings (herein also referred to as recessed ear holes) **111a/111b**, respectively.

The surface portion **113a** includes segments with varying slopes both along the downward direction toward the recessed ear hole opening **111a** as well as along a direction perpendicular to the downward direction. For example, as depicted schematically in FIG. **2C**, in this embodiment, the slope of the surface portion **113a** becomes steeper in a clockwise direction (looking from the top) such that a surface segment proximate the back surface **108** of the top surface has a steeper downward slope than a surface segment proximate the left side surface **105** of the top section, and a surface segment proximate the ridge **110** separating the left and the right portions has a steeper slope than the surface segment proximate the back surface **108** of the top section. Further, as noted above and discussed in more detail below, the slope of the surface portion **113a** in a direction orthogonal to the downward direction also exhibits variations at different locations of the surface. In this embodiment, the surface portion **113b** of the right head-receiving portion (cavity) is a mirror image of the left surface portion **113a** relative to an orthogonal plane bisecting the ridge separating the left and the right portions.

In other words, in this embodiment, the surface of each of the head-receiving portions **112a/112b** has a compound slope with variations in both downward direction and a direction orthogonal to the downward direction.

In this embodiment, each of the recessed ear openings **111a/111b** extends from the top surface **102** to the bottom surface **104** of the top section. In this embodiment, the recessed ear openings **111a/111b** have generally cylindrical shapes, with a generally circular cross-sectional profile, with lateral surfaces **120a/120b**, each of which extends from the top surface **102** of the top section to the bottom surface **104** thereof and has a convex curved profile. In other embodiments, the recessed ear openings can have other shapes. For example, in some embodiments, the recessed ear openings can have an elliptical cross-sectional profile. The recessed ear holes in combination with the sloped planes of the head-receiving surface help the user position their head in the top surface.

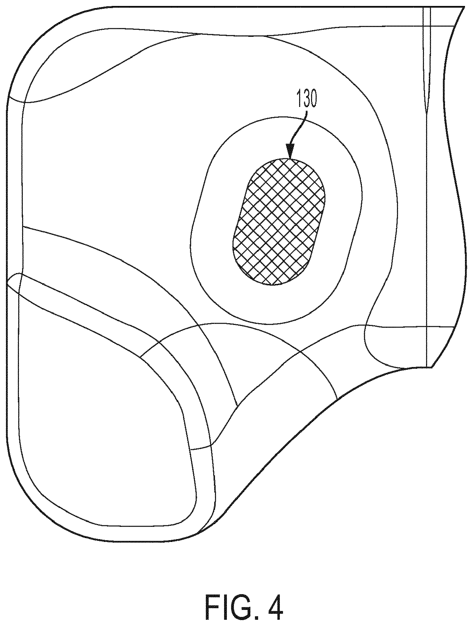

In some embodiments, a maximum cross-sectional dimension, e.g., a diameter, of each recessed ear opening can be, for example, in a range of about 1 inch to about 5 inches so as to comfortably accommodate the variable ear sizes of the users. As depicted in FIG. **4**, in some embodiments, at least a portion of the lateral surfaces of the ear openings **111a/111b** can be covered with a venting material **130.** Some examples of suitable venting materials include, without limitation, silk, cotton, wool, polyester or any combination thereof.

In addition to at least partially receiving a user's ear, the recessed ear openings can also muffle noise generated by the pillow which is experienced by the user while the user's head is maintained in one of the head-receiving portions,

With reference to FIGs. **2A**, **2B**, **2C** and **2D** and as noted above, the top section **101** further includes a left chin support **116a** and a right chin support **116b**, each of which protrudes above the surfaces **113a/113b** of the left and the right head-receiving portions **112a/1112b**, respectively. In this embodiment, the chin supports **116a/116b** include top surface segments **118a/118b** and lateral surface segments **117a/117b**, respectively, which extend from the top surface segments downwardly to the head-receiving portions (cavities) **112a/112b.**

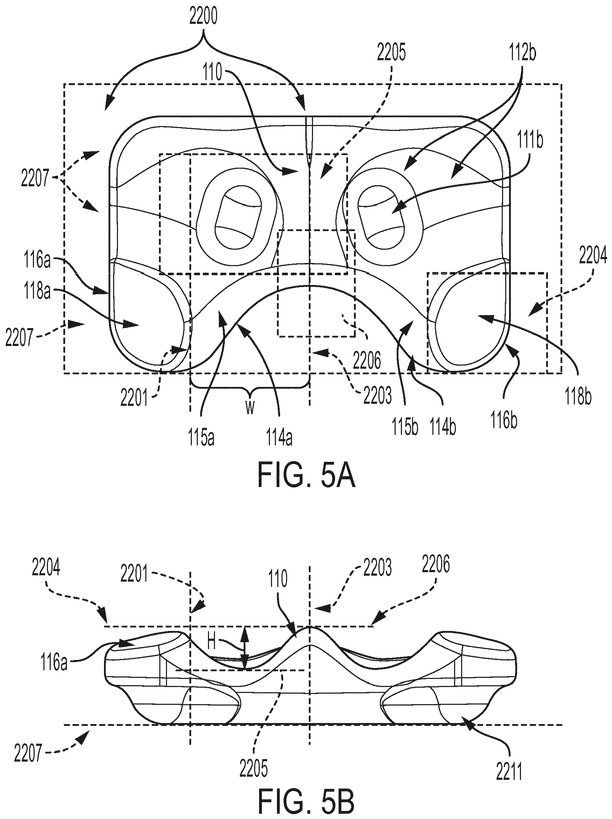

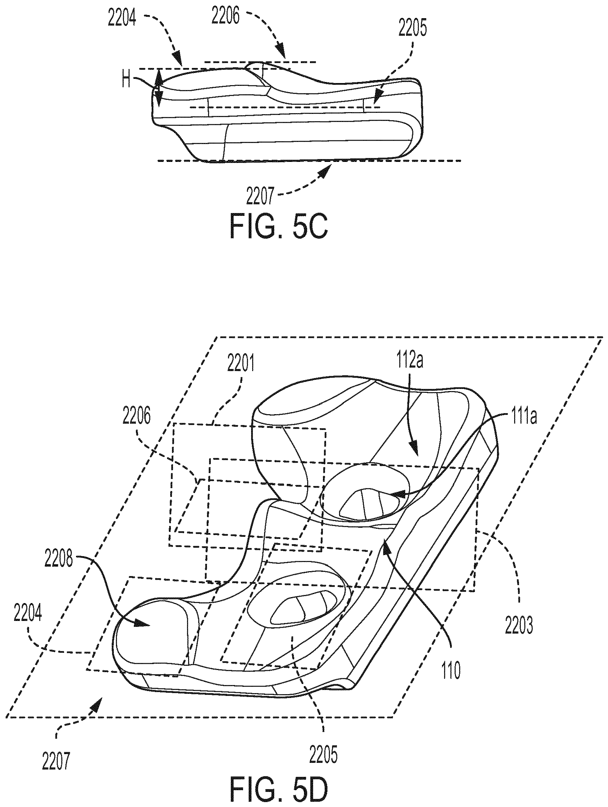

With reference to FIGs. **5A**, **5B**, **5C** and **5D**, a maximum height difference (H) between the top surface of each chin support **118a/118b** relative to the bottom of the respective head-receiving portions **112a/112b** can be, for example, in a range of about 1 inch to about 5 inches, e.g., in a range of about 1 inch to about 4 inches, e.g., in a range of about 2 to about 3 inches. In this embodiment, the maximum height difference between the top of a chin support and the bottom of a respective head-receiving portion can be determined as the distance between a putative plane **2205**, which is tangential to the lowest point of a head-receiving portion, which can cover in this embodiment the top of a respective recessed ear opening (e.g., the head-receiving portion **112b** covering the top of the recessed ear opening **111b**), and a putative parallel plane **2204** that is tangential to the highest point of the top surface of a chin support (e.g., the chin support **116a**) and parallel to a flat support surface **2207.**

FIGs. **5A**, **5B**, **5C**, and **5D** present other putative planes for ease of description of the relationships of various elements of the top section of the pillow **100** relative to one another. For example, the plane **2203** bisects the raised ridge **110.** The plane **2201** is parallel to the plane **2203** and is tangential to a portion of the inner lateral surface of the left chin block **116a** at the intersection of this lateral inner surface with the left recess neck opening **114a.** A putative plane similar to the plane **2201** can be defined with respect to the right chin support **116b.** The distance between the planes **2201** and **2203** can be defined as the width (W) of the left shoulder-receiving recess **115a.** The width of the right shoulder-receiving recess **115b** can be similarly defined. Further, the depth of each shoulder-receiving recess can be defined as a normal distance between the front tip of the ridge **110** (depicted as point P in this illustration) and a surface that is perpendicular to the support surface **2207** and is tangent to the front surface of the chin supports **116a** and **116b.**

With continued reference to FIGs. **5A**, **5B**, **5C**, and **5D**, the height (H) of the highest point of the ridge **110** separating the left and the right portions of the top section of the pillow **100** can be defined as the distance between a plane **2206** tangent to that point and parallel to the flat support surface **2207** and the plane **2205,** which is tangent to the lowest point of a head-receiving portion. The heights of other points along the ridge can be found in a similar fashion.

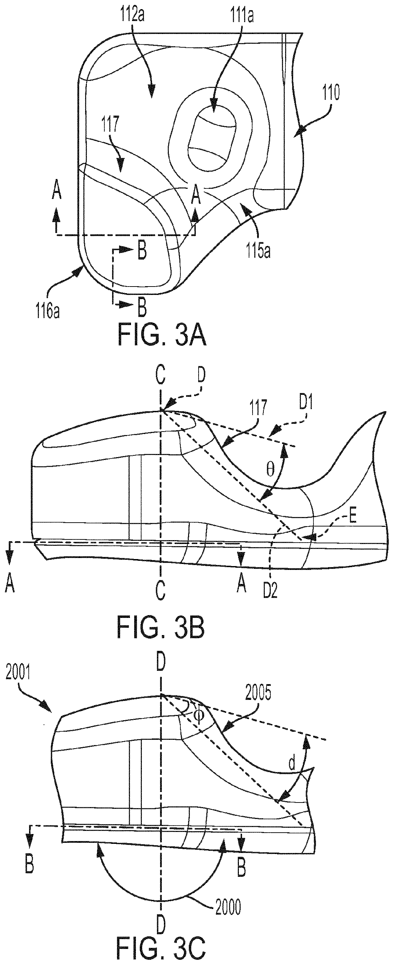

With continued reference to FIGs. **2C**, **2D**, **3A**, and **3B**, the lateral surfaces of the left chin support and the right chin support **116a/116b** include outer lateral surface segments **119a** and **119b** and inner lateral segments (herein also referred to as inner laterals surfaces) **117a/117b**, where the inner lateral segment of the left chin support extends from the front end of the top section in proximity of the left recess neck opening **114a** to the left side of the top surface, and the inner lateral segment of the right chin support extends from the front end of the top section in proximity of the right recess neck opening **114b** to the right side of the top section. The inner lateral segments of the chin supports exhibit a varying downward slope from the front end of the top section to a side thereof. For example, the inner lateral surface **117a** of the left chin support exhibits a downward slope that becomes progressively steeper as the inner lateral surface **117a** extends from the front end of the top section to its left side. The inner lateral segment **117b** of the right chin support is a mirror image of the inner lateral segment **117a** of the left chin support, and exhibits a progressively steeper downward slope as it extends from the front end of the top section to the right side thereof. In this embodiment, the compound slope of the inner lateral surface of each chin support is configured to substantially conform with the contour of the neck of individuals within 50^{th} percentile of the population,

As noted above, the chin supports **116a/116b** further include outer lateral surface segments **119a** and **119b**, respectively. The outer lateral surface segment **119a** of the left chin support **116a** is in the form of a curved surface that extends from the left end of the left recess neck opening **114a** to a lower end of the left side of the left head-receiving portion **112a.** Further, the outer lateral surface segment **119b** of the right chin support, which is a mirror image of the outer lateral surface **119a** of the left chin support, is also in the form of a curved surface that extends from the right end of the right recess neck opening **114b** to the lower end of the right side of the right head-receiving portion **112b.** In this embodiment, the outer lateral surfaces **119a/119b** of the left and the right chin supports **116a/116b** extend orthogonally from the top surface of the chin support to the bottom surface of the top section.

As noted above, the inner lateral surface of each of the left and the right chin support **116a/116b** has a compound slope that can vary across the inner lateral surface. By way of illustration, FIG. **3A** is a schematic plan view of the left portion of the top section of the pillow **100**, which depicts the head-receiving cavity **112a**, the chin support **116a** and the recessed ear hole **111a.** In this embodiment, an inner lateral surface **117** of the chin support **116a** exhibits a compound slope. For example, FIG. **3B** provides a cross-sectional view of the chin support **116a** along the A-A direction, This cross-sectional view depicts the downward slope of the chin support at a point (D) at the top of the chin support, which can be characterized by an angle (θ) between a putative line segment D1 tangent to the point (D) and a putative line segment D2, which extends from the point D to a point E, which is at the boundary of the inner lateral surface **117** of the chin support and the surface of the head-receiving cavity. As this figure shows, the slopes at other points along the inner lateral surface defined similarly as that defined for point (D) can vary from one surface point to another. Further, FIG. **3C**, which is a cross-sectional view of the inner lateral surface of the chin support along the B-B direction, shows that the slope of the inner lateral surface of the chin support varies also along a direction perpendicular to the downward direction. More specifically, FIG. **3C** shows that such a slope at the point D can be characterized by an angle ϕ, which is similarly defined as the angle θ above. Similar to the downward slope, such a slope of the lateral surface of the chin support can also vary from one point to another. Further, at each point of the lateral surface, the angles θ and ϕ can be the same or different.

The compound slope of the inner lateral surface of the chin support can help place a user in a sniff position, thus aligning the user's airways for improved airway management. In particular, in many embodiments, the compound slope of the inner lateral surface of the chin support can be the mirror image of the compound slope of the neck and platysmal surface of the side of the user's neck allowing the inner surface to comfortably receive said lateral platysmal surface.

In this embodiment, the top of each chin support is slanted toward a side of the top section of the pillow (i.e., to the left side for the left chin support and to the right for the right chin support). In other embodiments, the top surface of at least one, or both chin supports, can be flat.

As noted above and shown in FIG. **2D**, the top section **102** includes the ridge or raised surface **110**, which separates the left and the right portions of the top section **101** and assists in aligning a user's head and prevents the user from inadvertently assuming the supine position. In some embodiments, the ridge **110** rises above the surface of the head-receiving portions **112a/112b** such that it has a maximum height, as defined above and in connection with FIGs. **5A-5D**, in a range of about 1.5 inches to about 5 inches, e.g., in a range of about 2 inches to about 4.5 inches, or in a range of about 3 inches to about 4 inches, above the lowest portion of that surface.

With reference to FIG. **2B**, in this embodiment, the ridge **110** separating the left and the right portions of the top section of the pillow has a non-uniform height. In particular, the height of the ridge decreases as the ridge extends from the point (P) proximate the front surface of the top section to point (Q) proximate the back surface of the top section. In some such embodiments, the ridge **110** exhibits a height non-uniformity in a range of about 10% to about 300%, In other words, the fractional height difference between the highest and the lowest points of the ridge, as measured relative to the putative plane covering a recess ear opening, can be in a range of about 10% to about 300%.

The non-uniform: height of the ridge can help inhibit the user from inadvertently assuming a supine position while ensuring that the user will not experience claustrophobia due to excessive height of the ridge, particularly in the distal end of the ridge, which may block the user's vision.

Further, in some embodiments, the ridge extends at its proximal end from the right end of the left recess neck opening to the left end of the right recess neck opening. Referring to FIG. **2B**, the width (WR) of the ridge **110** at its proximal end can be, for example, in a range of about 3 inches to about 6 inches, e.g., in a range of about 4 inches to about 5 inches. The left and the right side surfaces of the ridge **110** slope downwardly to the left and right head-receiving portions **116a** and **116b.**

With reference to FIGs **2D**, the left and right recess neck openings **114a/114b** are in the form of curved ridges that extend from the left and right chin supports, respectively, to the ridge **110.** In this embodiment, each of the recess neck receiving ridges exhibits a varying radius of curvature from its one end to the other. For example, the radius of curvature of the left recess neck opening **114a** can increase as the recess neck opening extends from the chin support **116a** to the ridge **110**, and the radius of curvature of the right recess neck opening **114b** can decrease as the recess neck opening extends from the ridge **110** to the right chin support **116b.** In some embodiments, the recess neck openings can have a compound curvature, for example, in a range of about 1 inch to about 4 inches in order to able to comfortably receive differing user neck sizes. In other embodiments, one or both of the recess neck openings can be configured to have a single radius of curvature.

The dimensions of the shoulder-receiving recesses are generally chosen so as to inhibit the movement of a user from a lateral decubitus position to a supine position, For example, in this embodiment, the ratio of the width to the depth of each of the shoulder-receiving recesses **115a/115b** can be, for example, in a range of about 1.5:1 to about 6:1, e.g., in a range of about 4:1 to about 6:1. By way of example, the width of each of the shoulder-receiving recesses can be in a range of about 6 inches to about 18 inches, and the depth of each of the shoulder-receiving recesses can be in a range of about 1 inch to about 12 inches.

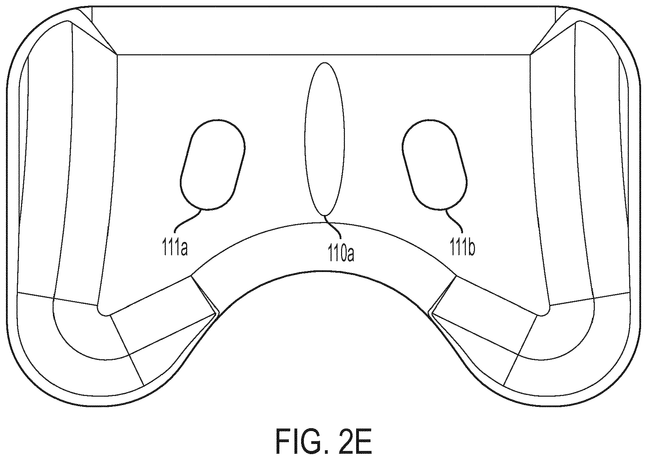

FIG. **2E** schematically depicts the bottom surface **104** of the top section, which can matingly engage with the top surface of the bottom section, depicting the bottom of each recessed ear hole **111a/111b** as well as a cavity **110a** associated with the ridge **110**, which separates the right and left portions of the top section.

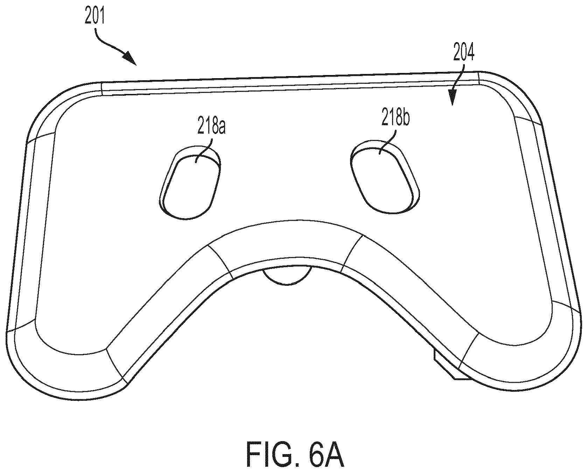

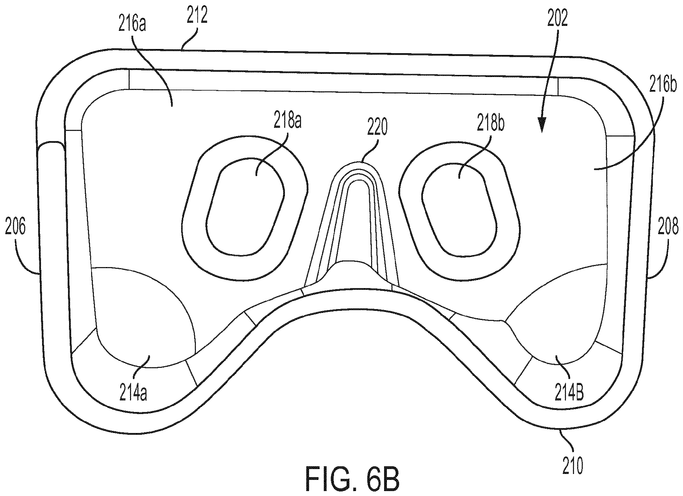

More specifically, with reference to FIGs. **6A** and **6B**, the bottom section **201** extends from a top surface **202** to a bottom surface **204.** The bottom section **201** further includes a left side surface **206**, a right side surface **208**, a front surface **210** and a back surface **212.** In this embodiment, the top surface **202** of the bottom section is shaped so as to matingly engage with the bottom surface of the top section.

In this embodiment, the bottom surface of the bottom section is substantially flat for positioning on a supporting surface, e.g., a mattress.

The bottom section **201** of the pillow can be removably and replaceably engaged with the top section **101** thereof. For example, as noted above, the top surface of the bottom section can be matingly engaged with the bottom surface of the top section. For example, as shown in FIG. **6B**, the top surface **202** of the bottom section **201** includes left and right raised ridges **214a/214b**, each of which is shaped and dimensioned to fit snugly within a pocket associated with a respective one of the chin supports **116a/116b** of the top surface **102** of the top section **101.** The top surface of the bottom section further includes a ridge **220** that is shaped and dimensioned to fit snugly within a cavity associated with the ridge **110** in the top section, which separates the left and right portions of the top section. Further, the top surface **202** of the bottom section **201** includes downward-sloping portions **216a/216b** that extend downwardly to recessed ear holes **218a/218b**, The downward-sloping portions **216a/216b** can fit matingly to portions of the back surface of the top section forming the back surfaces of the head-receiving portions **112a/112b** formed in the top section.

The recessed ear holes **218a/218b** formed in the bottom section of the pillow extend from the top surface of the bottom section to a bottom surface thereof. Similar to the recessed ear holes formed in the top section of the pillow, in this embodiment, the recessed ear holes **218a** and **218b** are substantially cylindrical with a convex-shaped lateral surface, though in other embodiments other cross-sectional profiles and shapes can be employed. The recessed ear holes **218a** and **218b** are positioned in the bottom section of the pillow such that upon coupling the bottom section with the top section of the pillow, each of the recessed ear holes in the bottom section is substantially aligned with a respective recessed ear hole in the top section. Thus, in this embodiment, when the top and the bottom sections of the pillow are engaged, recessed ear holes extend from the top surface of the top section of the pillow to the bottom surface of the bottom section of the pillow.

With reference to FIG, **4**, in some embodiments, the lateral surfaces of the recessed ear holes can be covered at least partially with a venting material **130.** By way of example, the venting material **130** can be in the form of a mesh. Some examples of suitable venting materials include, without limitation, silk, cotton, wool, polyester or any combination thereof.

The top and the bottom sections of the pillow can be formed of a variety of different materials. The material from which the top and the bottom sections of the pillow are fabricated is preferably hypoallergenic. Some examples of suitable materials can include, without limitation, polyurethane, a memory foam, an open cell foam, polyethylene and ethylene vinyl acetate (EVA). For example, the top and the bottom sections of the pillow can be formed of viscous elastic foam material. The foam material can be convoluted or otherwise configured to evenly distribute pressure caused by pressure points of the user's head and neck. By way of example, the convolutions of the foam material may exhibit a maximum depth of less than about 2 centimeters.

In some embodiments, the density of the material used to fabricate the pillow can be, for example, in a range of about 1.25 to about 1.35 lb/ft³. In some embodiments, different portions of the pillow can be fabricated using foam materials with different densities.

In some embodiments, the top section and the bottom section of the pillow can be fabricated using foam materials of different densities. For example, in some embodiments, the bottom section of the pillow can have a hardness greater than that of the top section of the pillow. By way of example, the difference in the hardness of the top and the bottom sections of the pillow can be characterized by an IDL equal to or greater than about 0.5, e.g., in a range of about 0.5 to about 2.

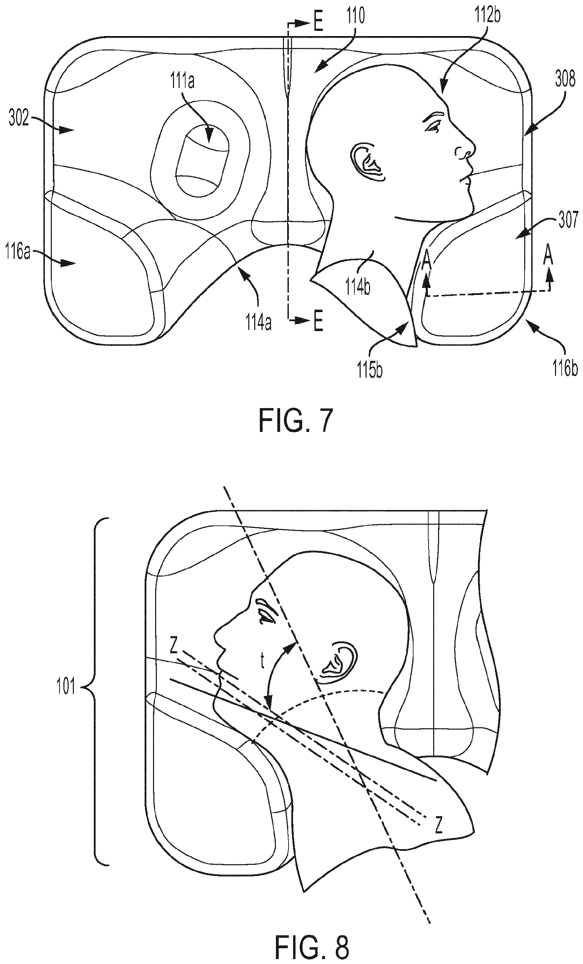

The pillow **100** can be used by a user in left lateral decubitus position and in the right lateral decubitus position. By way of example, FIG. 7 schematically depicts a user using the pillow **100** lying on his left side in a left lateral decubitus position with the user's head received and supported in the right head-receiving portion **112b** and the shoulder and the neck of the user supported by the right shoulder-receiving recess **115b** and the right recess neck opening **114b** allowing comfortable assumption of the lateral decubitus position. A portion of the lateral surface of the right chin support **116b** is in contact with a portion of the user's jaw to elevate the user's chin so as to cause extension of the user's upper cervical spine and a flexion of the user's lower cervical spine, thereby facilitating the placement of the user in a sniff position.

As discussed above with reference to FIG. **8**, in a sniff position, the laryngeal, tracheal and oropharyngeal airway axes of the head are better aligned than in a neutral position of the head, thus enhancing airway management. For example, in many embodiments of a pillow according to the present teachings, the pillow allows aligning the tracheal and laryngeal axes such that an angle between these two axes is less than about 10 degrees, and preferably less than about 5 degrees. Further, the pillow allows positioning of the user in a lateral decubitus position such that an angle between the oropharyngeal and laryngeal axes is less than 90 degrees, e.g., in a range of about 5 degrees to about 30 degrees.

In some embodiments, the pillow allows a user to assume the lateral decubitus position with the user's Occipito-Atlanto-Axial joint at an angle t between about 5 degrees and 60 degrees.

As discussed above, the left and the right chin supports facilitate the placement of the subject in a sniff position by elevating the subject's head. In many embodiments, each of the head-receiving portions, and the respective recess neck opening as well as the chin support are configured, dimensioned and positioned relative to one another such that when the user is in a lateral decubitus position with the user's head received by the head-receiving portion, the user's upper cervical spine experiences an extension in a range of about 5 to about 20 degrees and the user's lower cervical spine experiences a flexion in a range of about 5 to about 15 degrees,

Further, in some embodiments, the configuration and relative positioning of the head-receiving portion and the recess neck opening are such that the user's cervical spine can be maintained in substantially parallel alignment with the surface upon which the user is lying.

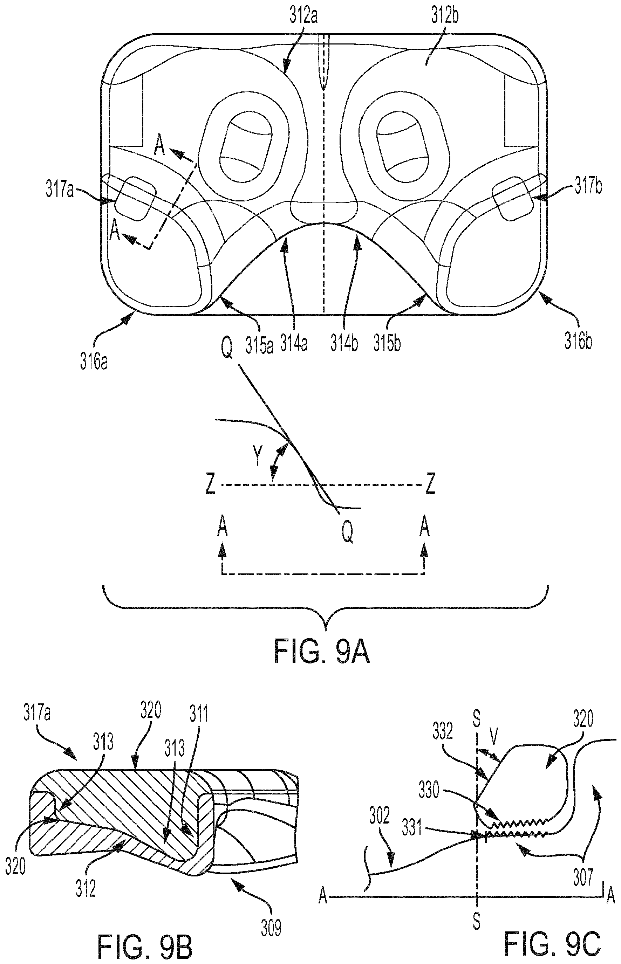

With reference to FIG. **9A**, in some embodiments, a pillow **300** can include a left chin support **316a** and a right chin support **316b**, where the left chin support **316a** includes an adjustable chin positioner **317a** and the right chin support **316b** includes an adjustable chin positioner **317b.** Similar to the previous embodiment, the pillow **300** includes a top section **301**, a bottom section (not shown in this figure), where the top section includes left and right portions having head-receiving portions **312a/312b**, recess neck openings **314a/314b**, and shoulder-receiving portions **315a/315b.** Similar to the previous embodiment, in this embodiment, each of the chin support **312a/312b** is in the form of a protrusion rising above a top surface **302** of the top section of the pillow. However, in this embodiment, each of the chin supports includes a movable/adjustable chin positioner that can be moved so as to accommodate different sizes and/or contours of the jaws, the chins and the necks of different users.

An adjustable chin positioner according to the present teachings can be implemented in a variety of different ways. For example, an adjustable chin positioner can be movably mounted onto a surface of a chin support using hook & loop fasteners, posts and corresponding holes, or other non-permanent means of attachment, which would allow moving the adjustable chin positioner to configure the pillow for a particular user.

By way of another example, with reference to FIG. **9B**, the adjustable chin positioner **317a** can include a chin block **320** that is movably engaged within a groove **313** provided on a top surface of the chin support **316a.** More specifically, as shown in FIG. **9C**, in this embodiment, the bottom surface **330** of the chin block **320** includes a sawtooth surface and the groove includes a mating sawtooth surface **331** for releasably engaging the sawtooth surface of the adjustable chin positioner such that the adjustable chin positioner can be moved back and forth along the groove. In this embodiment, the groove has a curved profile and extends from a front side of the top surface to a lateral side thereof. Further, in this embodiment, the chin block **320** includes a slanted lateral surface **332**, which can make an angle V, e.g., in a range of about 20 degrees to about 90 degrees, with a vertical axis S-S. The lateral surfaces of the chin block **320** are in contact with the lateral surfaces **313** and **311** of the groove. While in some embodiments, the slanted lateral surface **332** can be flat, in other embodiments it can be sloped. For example, it can have a compound slope.

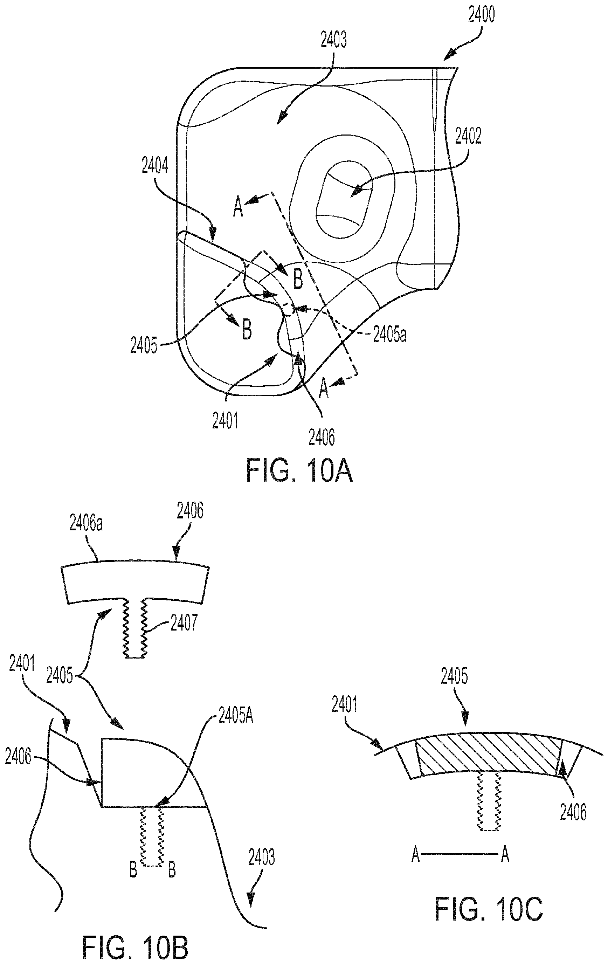

As noted above, the adjustable chin positioner can be implemented in a variety of different ways. By way of another example, FIGs. **10A**, **10B**, and **10C** schematically depict the left portion of a pillow **2400** that includes a head-receiving portion **2403** having a recessed ear hole **2402** at the bottom thereof, and a chin support **2401** having an inner lateral surface **2406**, which incorporates an adjustable chin positioner. More specifically, the inner lateral surface **2406** includes an opening **2405a** into which a chin support block **2405** can be movably coupled. In other words, the movable chin support block **2405** can be moved inward and outward relative to the opening so as to accommodate different sizes and contours of the jaws, the chins and the necks of different users.

With reference to FIG. **10B****,** in this embodiment, the chin support block **2405** of the adjustable chin positioner includes a collar **2406** from which a stem **2407** extends, where the stem can be movably positioned within the opening **2405a**. In this embodiment, a portion of the lateral surface of the chin support surrounding the opening **2405a** is recessed relative to the opening so as to allow swiveling the chin support block **2405** around the opening. In this manner, the chin support block **2405** is provided with two degrees of freedom, i.e.,in-and-out movement and rotation relative to the opening, which allow adjusting the chin support surface to a user's need. In this embodiment the outer surface **2406a** of the collar **2406** can have a curved profile such that when the support block **2405** is fully engaged within the opening **2405a**, the outer surface of the collar can be substantially flush, at least for one orientation of the collar, with the remainder of the lateral surface of the chin support. In some such embodiments, the outer surface **2406a** of the chin support block **2405** can exhibit a compound slope that can complement the compound slope of the inner lateral surface of the chin support.

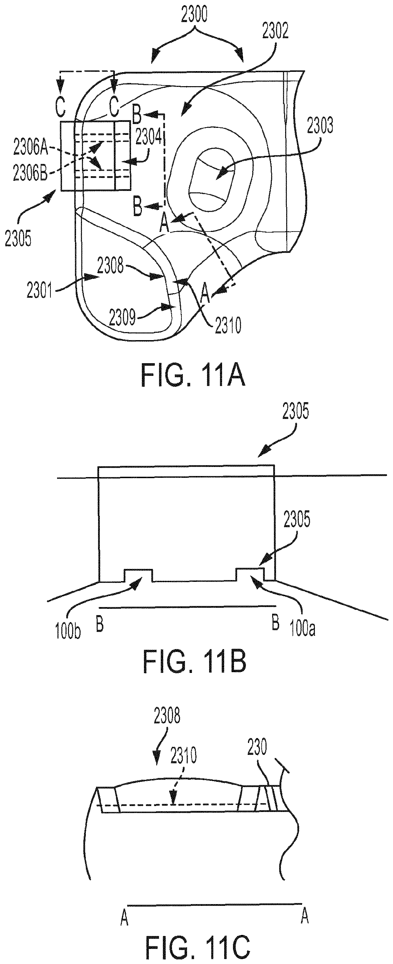

In some embodiments, the top section of a pillow according to an embodiment of the present teachings can include a left and a right removable and replaceable portion (block) that can be removed to allow access to the user's nose and mouth when the user's head is received in the left and the right head-receiving portions, respectively. By way of illustration- FIGs. **11A**, **11B**, and **11C** schematically depict the left portion of such an embodiment **2300**, which includes a head-receiving portion **2302** having a recessed ear hole **2303** at the bottom thereof. A chin support **2301** rises above the heard-receiving portion. The depicted pillow further includes a removable and replaceable block **2305**, which includes two grooves **100a/100b** for engaging with two rails **2306a** and **2306b** provided on the top surface of the pillow. Such engagement of the grooves in the removable block with the respective rails allows replaceably removing the block from the pillow so as to provide access, e.g., to a user's mouth and nose. By way of example, such access may be utilized to connect the user to a monitoring device and/or a therapeutic device (e.g., CPAP) as the user's head is received and supported in the heard-receiving portion in a lateral decubitus position.

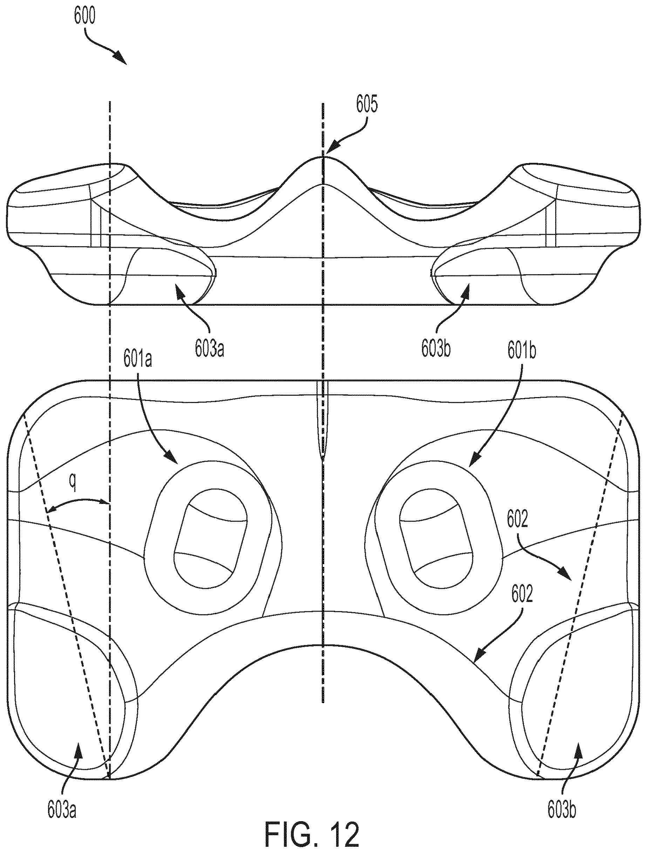

With reference to FIG. **12**, in some embodiments, the left and the right side surfaces of the top section of a pillow **600** according to an embodiment of the present teachings includes left and right cut-outs **603a** and **603b**, respectively, which can accommodate at least a portion of a user's left and right arms, as the user's head is received in the respective left and right head-receiving portions **601a/601b** in a left or a right lateral decubitus position. The arm cut-outs **603a/603b** provide additional comfort as the user uses the pillow in a left or a right lateral decubitus position. More specifically, FIG. **12** shows a plan view of the top surface of the pillow with spine or raised ridge **605** between head-receiving portions **601a/601b.** In this embodiment, the arm cut outs **603a/603b** are positioned at an angle q relative to axis x-x with angle z being in a range between 0° and 45°.

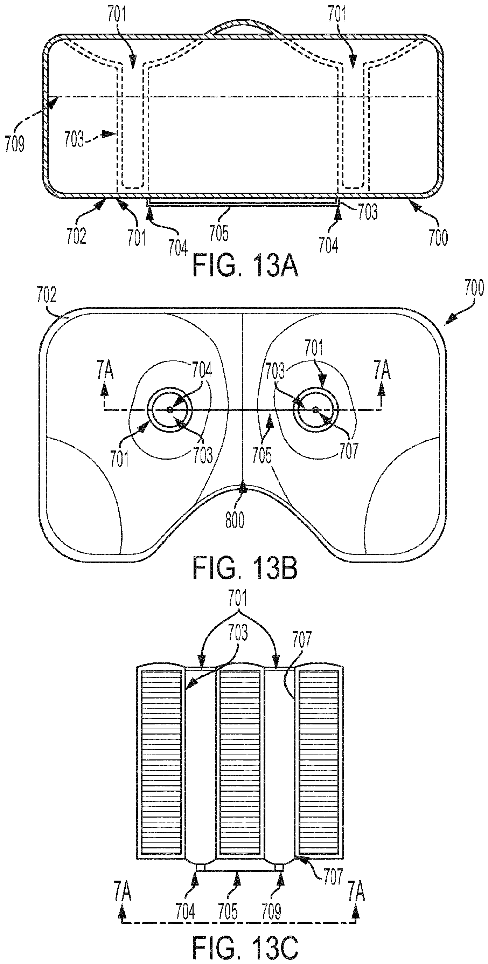

In some embodiments, in addition to the top section, the bottom section can also include respective arm cut-outs, e.g., similar to those shown for the top section. In some embodiments, such arm cut-outs provided in the bottom section can be positioned substantially below the respective arm cut-outs on the top section when the top and bottom sections of the pillow are engaged with one another. In some embodiments, a pillow according to the present teachings can be at least partially covered by a cover. By way of example, with reference to FIG **13A**, **13B**, and **13C**, a pillow **700** includes a top and bottom section (in this figure only the top section is visible). Further, similar to previous embodiments the top section includes a left and a right portion separated by ridge **800** where each of the left and right portions includes a head receiving portion, a chin support, and a recessed ear opening. For example, FIG **13A**, **13B**, and **13C** show how an embodiment of the cover can fit around a pillow. A zipper **709** in cover **702** allows pillow **700** to be inserted into cover **702** and be encapsulated therein, where cover **702** fits snugly over pillow **700.** Ear hole extension **703** can be fitted into, and passed down through, recessed ear hole cover **701** in pillow **700.** The end of each hole extension **703** distal to the top surface of pillow **700** in this embodiment has strap **705** attached to it by mechanical means (including sewing or glue). One end of strap **705** has a mechanical means of attachment (a button, hook, or hook and loop fabric) **704.** The end of ear hole extension **707** distal to top surface of pillow **700** in this embodiment has a means of receiving an attachment built into it (such as a button hole, a hook receptor, or hook and loop fabric). One end of strap **705** can then be attached to the end of ear hole extension 707 by these means of attachment and thus secure cover **702** around pillow **700.**

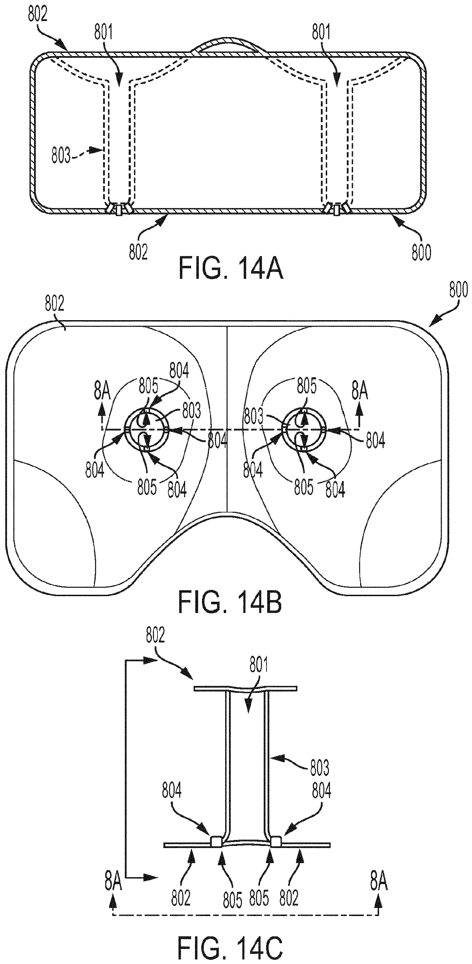

FIG **14A** shows a side view of another embodiment of a top section of a pillow **800** with a pillow cover **802** that fits snugly around pillow **800,** In this embodiment, the pillow cover **802** includes extension covers **803** that fit in through the recessed ear holes **801.** FIG. **14B** is a plan view of the bottom surface of top section of pillow **800** with cover **802** fitting snuggly around pillow **800** and the circumference of the bottom sides of recessed ear holes **801.** In this embodiment, the recessed ear hole extension covers **803** can include buttons **805**, which can be attached to the extension covers using a variety of different mechanisms (e.g., they can be sewn or glued to the extension covers). Further, portions of the bottom side of the cover surrounding the recessed ear holes **801** can include receptacles **804** for receiving the buttons, thereby securing the cover around the pillow. FIG. **14C** shows cutaway view of axis A-A (FIG. **14B**) in which recessed ear hole extension covers **803** with buttons **805** extend into, and through, the recessed ear holes **801** and are attached to cover **802** using the buttons **805.**

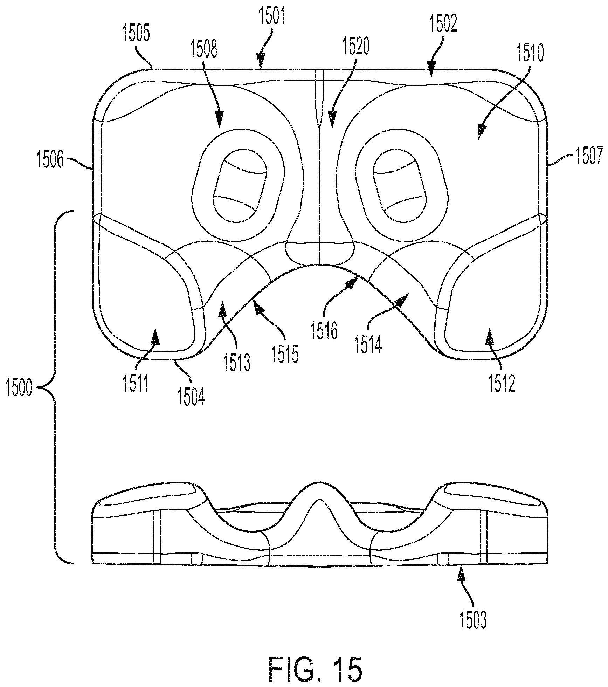

Although in this embodiment, the above apparatuses for airway management include two pieces, i.e., a top section and a bottom section, that can be removably and replaceably engaged with one another, in other embodiments, an apparatus according to the present teachings for airway management can be formed of a single piece. By way of example, FIG. **15** schematically depicts an apparatus **1500** for airway management that is formed as a substantially rectangular-shaped block **1501**, which extends axially between a top surface **1502** and a bottom surface **1503** and laterally between a front surface **1504**, a back surface **1505**, a left side surface **1506** and a right side surface **1507.** The apparatus **1500** is similar in all respects to the top section of the apparatus **100** discussed above, except that the bottom surface **1503** of the apparatus **1500** is substantially flat so as to allow positioning it on a flat surface. In particular, the apparatus **1500** includes left and right head-receiving portions **1508/1510**, left and right chin supports **1511/1512**, left and right recess neck openings **1513/1514**, left and right shoulder-receiving recesses **1515/1516**, and a raised ridge **1520**, which separates the left portion from the right portion.

Thus, similar to the previous embodiments, the pillow **1500** allows placing a user in a left or a right lateral decubitus position with the user's head received and supported in the head-receiving portions and user's neck and shoulders supported by the recess neck openings and the shoulder-receiving recesses.

Those having ordinary skill in the art will appreciate that various changes can be made to the above embodiments without departing from the scope of the invention.

## Claims

1. An apparatus for supporting the head and neck of a user for airway management, comprising:
a top surface including at least one head-receiving portion configured and dimensioned for receiving and supporting a user's head, and at least one recess neck opening for supporting a user's neck when the user's head is received in said head-receiving portion, and
at least one chin support protruding above said top surface and configured for facilitating placing the user in a sniff position when the user's head is received in said head-receiving portion,
said chin support comprising a top surface segment and a lateral surface segment,
wherein at least a portion of the lateral surface segment of the chin support extends from said top surface segment thereof to said at least one recess neck opening.

2. The apparatus of claim 1, wherein said head-receiving portion, said recess neck opening and said chin support are positioned relative to one another and dimensioned such that when the user is in a lateral decubitus position with the head received by the head-receiving portion, oropharyngeal, laryngeal and tracheal axes are substantially aligned.

3. The apparatus of claim 1, wherein said head-receiving portion, said recess neck opening and said chin support are positioned relative to one another and dimensioned such that when the user is in a lateral decubitus position with the user's head received by the head-receiving portion, the user's upper cervical spine experiences an extension in a range of about 5 to about 20 degrees and the user's lower cervical spine experiences a flexion in a range of about 5 to about 15 degrees.

4. The apparatus of claim 1, wherein a maximum height difference between said top surface segment of the chin support and said top surface is in a range of about 1 inch to about 4 inches.

5. The apparatus of claim 1, wherein said at least a portion of the lateral surface segment of the chin support exhibits a downward slope toward said head-receiving portion, and wherein optionally said downward slope of said at least a portion of the lateral surface segment of the chin support varies in a range of about 90 degrees to about 20 degrees.

6. The apparatus of claim 1, wherein said top surface segment of the chin support is downwardly slanted toward a lateral side of said top surface.

7. The apparatus of claim 1, wherein said at least a portion of the lateral surface segment of the chin support exhibits a compound slope.

8. The apparatus of claim 1, wherein said neck recess opening comprises a concave surface portion, and wherein optionally said concave surface portion of the neck recess opening has a radius of curvature in a range of about 1 inch to about 4 inches.

9. The apparatus of claim 1, wherein said at least one head-receiving portion comprises a left and a right head-receiving portion separated by a ridge, and wherein said at least one recess neck opening comprises a left and a right recess neck opening separated by said ridge, and wherein said at least one chin support comprises a left chin support and a right chin support for facilitating placing the user in a sniff position where the user's head is received in said left and right head-receiving portions, respectively.

10. The apparatus of claim 1, wherein said chin support comprises an adjustable chin positioner, and wherein optionally said adjustable chin positioner comprises a groove and a chin support block configured to be movably received in said groove such that said chin support block is movable along said groove, and wherein optionally a bottom surface of said chin support block comprises a sawtooth surface and said groove has a mating sawtooth surface for engaging said sawtooth surface of said chin support block such that said chin support block can be moved along said groove.

11. The apparatus of claim 10, wherein said groove has a curved profile, and wherein said curved groove extends from a front side of said top surface to a lateral side thereof.

12. The apparatus of claim 10, wherein said adjustable chin positioner comprises:
an opening formed in a surface of the chin support, and
a chin support block movably positioned in said opening formed in the chin support such that the movement of the chin support block in and out of the opening can allow adjusting a chin support surface presented to the user.

13. The apparatus of claim 12, wherein said chin support block comprises a collar and a stem extending from said collar, and wherein optionally said collar has an outer surface exhibiting a compound slope.

14. The apparatus of claim 9, wherein said ridge has a maximum height in a range of about 1 inch to about 5 inches.

15. The apparatus of claim 9, wherein said ridge has a non-uniform height, and wherein optionally said ridge exhibits a greater height proximate a front side of said top surface relative to a back side of said top surface.

16. The apparatus of claim 15, wherein said non-uniform ridge exhibits a height non-uniformity in a range of about 10% to about 300%.

17. The apparatus of claim 9, wherein said ridge has a maximum width in a range of about 4 inches to about 6 inches.
